# EUROPEAN PATENT SPECIFICATION

(11) **EP 1 853 347 B1**
(45) Date of publication and mention of the grant of the patent: **11.04.2012**
(21) Application number: 06710427.3
(22) Date of filing: 17.02.2006
(51) Int. Cl.: A61N 5/06

(54) **LIGHT THERAPY DEVICE FOR TREATMENT OF BONE DISORDERS AND BIOSTIMULATION OF BONE AND SOFT TISSUE**
LICHTTHERAPEUTISCHE VORRICHTUNG ZUR BEHANDLUNG VON KNOCHENERKRANKUNGEN UND BIOSTIMULATION VON KNOCHEN UND WEICHTEILEN
DISPOSITIF DE PHOTOTHERAPIE POUR LE TRAITEMENT DES AFFECTIONS OSSEUSES ET LA BIOSTIMULATION DES OS ET DES TISSUS MOUS

(30) Priority: 16.02.2006 US 355583; 17.02.2005 US 653828 P; 05.08.2005 US 705753 P
(43) Date of publication of application: 14.11.2007
(73) Proprietor: Brawn, Peter Robert, Vancouver, British Columbia V6C 3R4 (CA)
(72) Inventor: Brawn, Peter Robert, Vancouver, British Columbia V6C 3R4 (CA)
(74) Representative: Boyce, Conor
(86) International application number: PCT/IB2006/000358
(87) International publication number: WO 2006/087633

(56) References cited:
- WO-A-2004/075985
- WO-A-2005/107637
- GB-A- 2 376 891
- GB-A- 2 416 311
- US-A- 4 244 373
- US-A- 4 457 707
- US-A- 4 840 174
- US-A- 5 421 727
- US-A- 5 616 140
- US-A1- 2003 009 205
- US-B1- 6 471 716

## Description

### FIELD OF THE INVENTION

The present invention relates to a light therapy device used for the treatment of bone disorders and the biostimulation of bone and soft tissue. The present invention is designed as an external device to be used on the jaw bone or other bones and soft tissues. One or more light emitting diode ("LED") arrays are used as the means for the treatment and biostimulation.

### BACKGROUND OF THE INVENTION

Osteonecrosis is the death of bone due to inadequate blood flow to the tissues. It is known by many other names including avascular necrosis or ischemic necrosis. Ischemic necrosis literally means "dead bone from poor blood flow." It includes dead bone or bone marrow that has been slowly strangulated or nutrient-starved. It occurs because of a decrease in blood supply to specific parts of bones. The decreased circulation causes cells in the bone and bone marrow to die. Bone with chronically poor blood flow develops either a fibrous marrow; a greasy, dead fatty marrow; a very dry, sometimes leathery marrow; or a completely hollow space. Osteonecrosis is usually seen in the jaw, hips, and knees although any bone may develop this disease. There are a number of local and systemic problems capable of producing this bone disease. However, research has shown that more than 4 out of every 5 patients with osteonecrosis have a problem, usually inherited, of excessive production of blood clots in the blood vessels (See, for example, A Note to Patients with Jawbone Osteonecrosis (NICO), available at http://maxillofacialcenter.com/NICOhome.html#note).

Anything leading to blocked blood vessels can cause osteonecrosis such as abnormal red blood cells as seen in sickle cell anemia. Additionally, taking high doses of corticosteroids or expanding nitrogen bubbles (decompression sickness as seen in scuba divers) may also lead to osteonecrosis. Osteonecrosis may have no signs or symptoms, but some people experience pain, especially when pressure is applied to the bone.

Although in some cases the bone may heal itself, the majority of patients who have osteonecrosis must seek the aid of a doctor. Common treatments include curettage of the bone lesion to remove the diseased bone marrow, combining surgery with antibiotic therapy, surgery with hyperbaric chamber therapy or anticoagulation therapies.

Light therapy is a treatment option which involves stimulation of a variety of biological activities in cells and tissues that are compromised in function. Optimally functioning cells and tissues are not stimulated by light therapy. Cells and tissues contain light sensitive proteins, chromophores and cytochromes, which have the ability to absorb light energy at specific wavelengths and to transform the light energy into chemical energy. In addition, specific wavelengths stimulate enzymatic activities that are in metabolic pathways of the mitochondria, increasing cellular energy. The cells and tissues then use the chemical energy to accelerate the natural healing processes of the body. One of the most frequent effects of light therapy is increased blood and lymphatic circulation in the area exposed to the light. Other effects include decreased pain and inflammation, accelerated new bone formation, and new blood vessel formation. In addition, there is a variety of cellular and membrane activity that is stimulated by specific wavelengths and energy densities.

Light therapy treatment devices currently are used to treat tissue disorders, such as pain and inflammation. The use of a light therapy treatment device can also effectively be used to treat bone disorders, such as jaw osteonecrosis or other jaw bone disorders. Light therapy treatment devices may also be used to stimulate bone formation, soft and hard tissues, as well as for the treatment of diseased bone or tissue.

Light therapy treatment devices currently exist which use laser light and discrete light-emitting diodes or LEDs as the source of light energy. A laser uses coherent light that emits a beam of photons at specific wavelengths. An LED emits incoherent monochromatic light at specific wavelengths. The LED array has a larger surface area for treatment due to the large number of diodes on the array. The intensity of the LED array is more diffuse than laser, thereby reducing potential damage to the eye. The use of multiple wavelength LEDs on the array allows for irradiation over multiple wavelengths for greater biological activity.

Light therapy treatment may be administered by the physician, therapist or patient through the use of a hand-held light emitting wand or a light emitting device placed on the affected area of the body intended for treatment. Light emitting wands and light emitting devices are difficult to position consistently over the affected area. Sometimes a tattoo is used to identify the affected area; however, due to the difficulty in consistent placement of these designs, the constant positioning is not easily attainable. The use of a light emitting wand or a light emitting device is not an accurate, consistent or repeatable method of light therapy treatment.

Using lasers and LEDs for treatment produces significant heat due to the thermal generating nature of the lasers and LED semiconductor. Due to this production of heat, the light therapy device gets hot, making it difficult to provide effective treatment as the device loses its efficiency and safety. Due to diminished LED efficiency in response to increases in operating temperature, current light therapy devices must be reduced or pulsed in order to keep the extra-oral LED array and surface of the device cool to provide comfort to the patient and to avoid potential burns. Most LED devices are of low intensity in an attempt to correct for the heat generation. Current light therapy devices are not effective at controlling the significant heat produced.

Most currently available light therapy devices are designed for use at a physician's, dentist's or therapist's office. Light therapy treatment requires repetition in order to effectively treat jaw osteonecrosis, other jaw bone disorders, periodontitis, orthodontics, or orthopedics, to stimulate and accelerate post-oral surgery or post- periodontal surgery healing, to accelerate osseo-integration of endosseous dental implants, and to treat and stimulate new bone formation and to treat and stimulate soft and hard tissues. Thus, patients may be required to make several visits to a practitioner's office or clinic in order to complete a therapy regimen. Such repeated visits may be time consuming and/or expensive.

U.S. Patent No. 6,471,716, Pecukonis discloses a phototherapy device that emits photo-therapeutic radiation to treat living tissue. Infrared light emitters may be incorporated into a mouthpiece for treating gum tissues.

In view of the above, there is a need or desire for a light therapy device having the ability to apply specific wavelengths of light to affected bone for treatment and stimulation of new bone formation and/or for the treatment and stimulation of soft and hard tissues.

There is also a need or desire for a light therapy device which can produce accurate, consistent and repeatable treatment results particularly in the dental and maxillofacial areas.

There is a further need or desire for a light therapy device which can be effectively administered against the affected area without resulting in pain from the production of heat and which provides more efficient and effective light therapy treatment by correcting for this heat generation.

WO 02/062419 A1 discloses an extra-oral light therapy device for the treatment of temporamandibular diseases comprising a head-set.

There is still a further need or desire for a light therapy device which can be used at home by the patient with a better and more secure fit.

### SUMMARY OF THE INVENTION

In accordance with the present invention there is provided an extra-oral light therapy device according to claim 1. The light therapy device is capable of consistently and reproducibly applying specific wavelengths of light to affected bone or tissue for treatment. Suitably, the light therapy device has been developed for clinical and/or in-home use.

In one embodiment, the extra-oral light therapy device may have a head-set style arrangement including a head-set, at least one extra-oral LED array removably connected to the head-set, a connector for removably attaching the at least one extra-oral LED array to the head-set, and a programmable controller.

In one embodiment of the present invention an external light therapy device includes at least one light emitting diode ("LED") array that is mounted on a thin, molded substrate, an attaching means for securing the external light therapy device to the area of treatment, and a programmable controller.

The device can be used for the treatment and stimulation of soft and hard tissue and the biostimulation of bone. This involves connecting at least one extra-oral LED array to the extra support, programming a controller to direct the at least one extra-oral LED array to emit pulsed or continuous incoherent monochromatic light, and emitting pulsed or continuous incoherent monochromatic light from the at least one extra-oral LED array. The controller is programmed to direct the extra-oral LED array to emit pulsed or continuous incoherent monochromatic light at predetermined rates, frequencies, intensities and durations according to a prescribed treatment regimen in order to stimulate and accelerate bone formation and healing at a select treatment area. The method may further include rotating the at least one extra-oral LED array between a sagittal axis and a vertical axis to affect the treatment area. Suitably, the extra-oral LED array emits light at wavelength between 820-890 ran and between 620-680 nm. Suitably, the programmable controller turns off the light when the level of heat produced by the light exceeds a set level.

Alternatively, the device can be used for treating and stimulating soft and hard tissues and biostimulating bone including attaching at least one LED array which includes at least one reflector to a treatment area, programming a controller to direct the at least one LED array to emit pulsed or continuous incoherent monochromatic light, emitting pulsed or continuous incoherent monochromatic light from the at least one LED array onto a treatment area, and focusing the emitted light onto the treatment area using the at least one reflector, wherein the pulsed or continuous incoherent monochromatic light stimulates and accelerates bone and soft tissue formation and healing within the treatment area. Suitably, the LED array may include at least one reflector and optic which focuses the at least one LED array at an angle of about 45° to about 60°; the extra-oral LED array emits light at wavelength between 820-890 nm and between 620-680 nm; and the programmable controller turns off the light when the level of heat produced by the light exceeds a set level.

These and other embodiments are more fully described in connection with the drawings and detailed description.

### BRIEF DESCRIPTION OF DRAWINGS

FIG 1 is a top view of a programmable controller.
FIG 2 is a perspective view of an embodiment of an extra-oral light therapy device with a head-set style arrangement, a head-set, at least one LED array, and a connector.
FIG 3 is a side view of an alternate embodiment of the extra-oral light therapy device with the head-set style arrangement, a head-set, at least one LED array, and a connector.
FIG 4 is a front-facing view of at least one LED array, and a connector detached from a head-set.
FIG 5 is a front-facing view of the external light therapy device with two LED arrays, a hinge-like member, and an attaching means.
FIG 6 is a cross-sectional view of an LED array mounted onto a substrate.
FIG 7 is a cross-sectional view of an LED array detached from a substrate.

### DETAILED DESCRIPTION OF THE INVENTION

The present invention relates to a light therapy device used for the treatment of bone disorders and the biostimulation of bone and soft tissue. The present invention is designed as an external device to be used on the jaw bone or other bones and soft tissues. One or more light emitting diode ("LED") arrays are used as the means for the treatment and biostimulation.

One embodiment of the present invention relates to a device having at least one extra-oral LED array supported by an extra-oral bridge, controlled by a programmable controller for the treatment of jaw osteonecrosis, other jaw bone disorders, periodontitis, orthodontics, or orthopedics, for stimulation and acceleration of post-oral surgery or post-periodontal surgery healing, and to accelerate osseo-integration of endosseous dental implants.

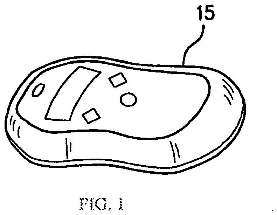

FIG 1 illustrates a programmable controller 15. The programmable controller 15 may be composed of a microprocessor and the associated electronic circuitry and suitably powers the present invention. Suitably, the circuitry in the programmable controller 15 monitors the changes in current/voltage and calculates the temperatures of the LED by using an algorithm programmed into the programmable controller software. A fail safe circuitry will shut off the current and light if the heat exceeds a pre-set level. A physician, dentist, or therapist may program a patient's treatment regimen into the programmable controller 15. The programmable controller 15 may control the energy density, pulse frequency and/or duration of light emitted by the extra-oral light therapy device. The programmable controller 15 may have pre-set programs built in, pre-defined by the physician, dentist, or therapist so that a patient is able to use the device under specific pre-programmed instructions. A patient can then utilize the extra-oral light therapy device at home through the use of the programmed treatment regimen in the programmable controller 15. The programmable controller 15 may be a separate, remote unit or may be directly connected to the present invention.

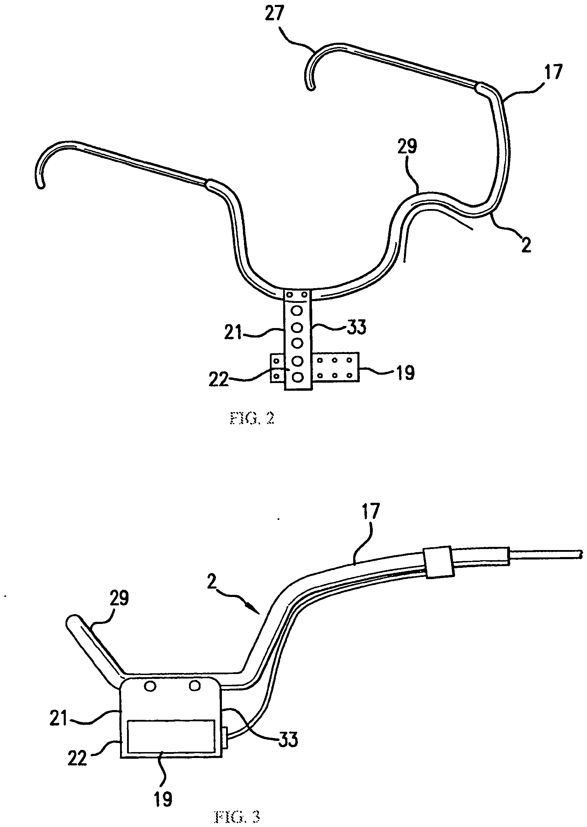

In embodiments of the invention, as shown in FIG 2 and FIG 3 the extra-oral light therapy device 2 may have a head-set style arrangement. The extra-oral light therapy device 2 includes a head-set 17, at least one extra-oral LED array 19, and connector 21. The head-set 17 can be modeled as a traditional pair of eyeglasses with form-fitting arms 27 that fit above and around the ears, and a frame 29 that fits on the bridge of the nose to secure the pair of glasses. The form-fitting arms 27 can be made of any firm, resilient material that allows for some flexibility for a better and more secure fit for individual users. The form-fitting arms 27 can also be adjusted horizontally along their axis. The frame 29 can also be adjustable to allow for a better and more secure fit The head-set 17 may also include lenses (not shown) like a traditional pair of eyeglasses. Suitably, the lenses may be made of a protective material to shield the patient's eyes from the LED array.

In another embodiment, the head-set 17 can be modeled as an adjustable strap (not shown) which fits around the crown of a patient's head for securing the extra-oral light therapy device 2. The adjustable strap can also fit around a patient's chin and extend back to the crown and around the crown of a patient's head. The adjustable strap is preferably made of a flexible, elastic woven material.

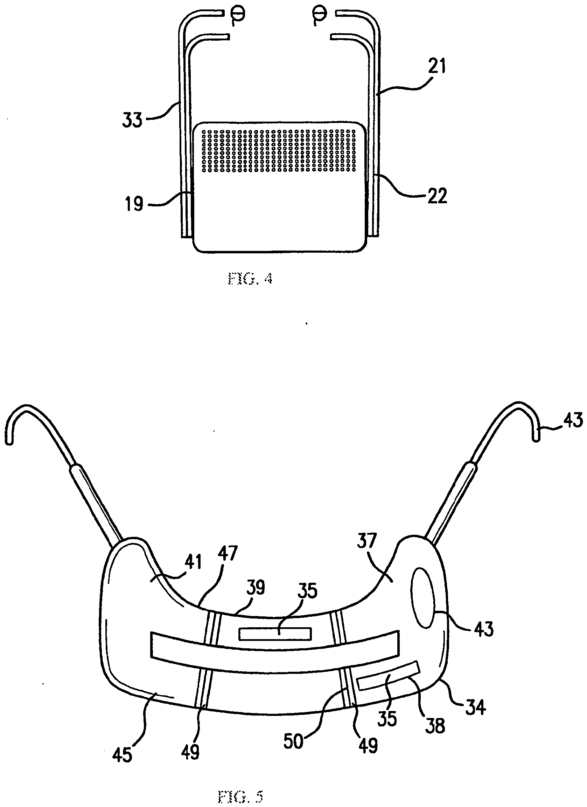

A connector 21 is attached to the head-set 17. A bar, rod or similar device 33 is fastened to the connector 21. The at least one extra-oral LED array 19 is then attached through a clip or similar mechanism 22 to the bar, rod or similar device 33. As shown in FIG 4, the at least one extra-oral LED array 19 can be removably detached from the head-set 17. The at least one extra-oral LED array 19 may be adjusted along a horizontal axis (not shown), relative to the head-set 17, or a vertical axis (not shown), relative to the head-set 17. The bar, rod, or similar device 33 may be comprised of a flexible but firm material sufficient to sustain the weight of the at least one extra-oral LED array 19.

The present invention also relates to a method of treatment for jaw osteonecrosis, other jaw bone disorders, periodontitis, orthodontics, or orthopedics, a method of stimulation and acceleration of post-oral surgery or post-periodontal surgery healing, and a method of acceleration of osseo-integration of endosseous dental implants. The method utilizes the extra-oral light therapy device. A physician, dentist, or therapist programs a patient's prescribed treatment regimen into the programmable controller 15. The programmable controller 15 controls the energy density, pulse frequency and duration of the extra-oral light therapy device. The programmable controller 15 runs a patient's prescribed treatment regimen causing the at least one extra-oral LED array to emit pulsed or continuous incoherent monochromatic light at the prescribed rates and frequencies onto the treatment area. Therefore, stimulating and accelerating bone formation and healing at a patient's treatment area for the treatment of jaw bone disorders and jaw osteonecrosis.

Another embodiment of the present invention relates to an external light therapy device 34 comprising at least one LED array 35 that is mounted on a thin, molded substrate 51, an attaching means 43 for securing the device to the area of treatment, and a programmable controller 15 for the treatment and stimulation of soft and hard tissue and the biostimulation of bone.

Referring to FIG 5, the external light therapy device 34 having a right section 37, a center section 39 and a left section 41 includes an LED array 35. FIG 5 illustrates the present invention for use for the treatment and stimulation of the jaw and facial bones and tissues, and fits around a patient's mouth, with the right section 37 and the left section 41 secured on the right and left sides of a patient's face with the attaching means 43. The attaching means 43 can be an adhesive such as double-sided adhesive tape or the attaching means 43 can also be form-fitting arms which surround the ear, such as those used in traditional eyeglasses, wherein such attaching means are designed for use in patients of differing facial sizes and to allow for flexibility for a more comfortable patient fit. Alternatively, the attaching means 43 can be utilized as an intra-oral means such as bite tabs or a tray device for proper positioning.

The external light therapy device 34 as shown in FIG 5 has at least one LED array 35 which is preferably permanently mounted on a thin, molded substrate 51. More than one LED array may be used in the device. For example, FIG 5 shows the device with two (2) arrays. The LED arrays may be arranged such that there is a lower level 45 and an upper level 47. The LED arrays may be removably attached through the use of one or more connectors 38 such as ribbon connectors. In between the LED arrays, a hinge-like member 49 is preferably integrated to allow for a more secure fit around the facial area. The hinge-like member 49 may be a thin crease 50 set into the substrate material, as illustrated in FIG 5. The hinge-like member 49 allows the center section 39 to fit around a patient's mouth and the right section 37 and the left section 41 to fit around a patient's face.

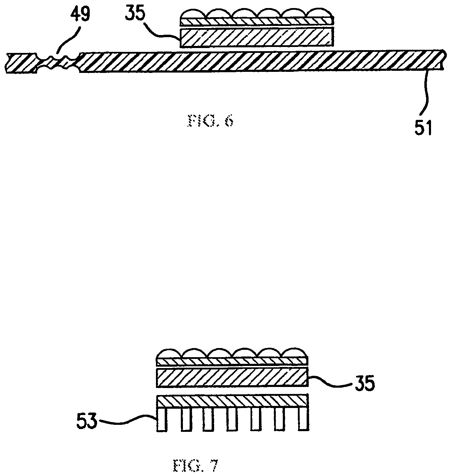

The at least one LED array 35 may be permanently mounted on a thin, molded substrate 51 as illustrated in FIG 6, FIG 7 shows a cross-section of the at least one LED array 35 of the external light therapy device 34 detached from the substrate 51. A clip or similar attaching means 53 allows the at least one LED array 35 to be mounted onto the substrate 51. The thin, molded substrate 51 is used as a heat sink as described above. The substrate 51 may be made of aluminum, copper or similar thermally conductive material to allow for a flexible, but somewhat rigid material. Optical focus from the LED array may be built into the array; reflectors and optics may be included as part of the LED array and these are suitably encapsulated in plastic or similar material and act to direct the light from the LED array. The optimal optical focus for the reflectors is approximately at an angle between 45-60°.

The at least one LED array 35 emits incoherent monochromatic light at varying frequencies and high intensity wavelengths. The light energy emitted from the LED array may be continuous or pulsed at predetermined rates and frequencies. The LEDs are arranged in a variety of patterns to achieve uniform optical density on the treatment area. The LEDs are suitably arranged in staggered parallel rows to maximize the number of LEDs on the LED array. Suitably, the LED array may emit light at wavelengths of between about 820 to about 890 nm and between about 620 to about 680 nm.

The present invention also relates to a method for the treatment and stimulation of soft and hard tissue and the biostimulation of bone. The at least one LED array 35 is first attached to the desired area of treatment. A physician, dentist, or therapist programs a patient's prescribed treatment regimen into the programmable controller 15. The programmable controller 15 controls the energy density, pulse frequency and duration of the light emitted from the external light therapy device 34. The programmable controller 15 runs a patient's prescribed treatment regimen causing the at least one LED array 35 to emit pulsed or continuous incoherent monochromatic light at the predetermined rates and frequencies onto the treatment area. The light therapy device features provide effective, stabilized, repeatable, accurate, programmable, and consistent light therapy for the treatment and stimulation of soft and hard tissue and the biostimulation of bone.

## Claims

1. An extra-oral light therapy device (2) for the treatment of jaw bone disorders and jaw osteonecrosis and biostimulation of bone and soft tissue, comprising:
an extra-oral support (5), the extra-oral support comprising a head set (17) and a frame (29) adapted to fit on the bridge of a patient's nose and to lie over the patient's face when the device is worn by the patient;
at least one extra-oral light emitting diode ("LED") array (4) that is removably connected to the frame via a connector (21), that is adapted to contact the patient's face on the patient's jawbone when the device is worn by the patient, and that is adapted to irradiate light through the patient's face to the patient's jawbone and soft tissue when the device is in use; and
a programmable controller (15) for controlling the extra-oral light therapy device.

2. The extra-oral light therapy device of Claim 1 wherein the head set comprises form-fitting arms (27) that conform to a patient's ears.

3. The extra-oral light therapy device of Claim 1 wherein the connector removably attaches the at least one extra-oral LED array (19) to the frame by clipping the at least one extra-oral LED array to a bar or rod (33) fastened to the connector.

4. The extra-oral light therapy device of Claim 1 wherein the at least one extra -oral LED array adjusts along a horizontal axis and a vertical axis.

5. The extra-oral light therapy device of Claim 1 wherein the at least one extra-oral LED array comprises a plurality of thick-film ceramo-metal LED wafers and a metal substrate for transferring heat produced to an aluminum heat sink.

6. The light therapy device of Claim 1 wherein the programmable controller is programmed with a patient's prescribed treatment regimen.

7. The light therapy device of Claim 1 wherein the programmable controller comprises circuitry configured to monitor changes in current or voltage.

8. The light therapy device of Claim 7 wherein the controller comprises a fail-safe circuitry that shuts off current and light if heat in the devi ce exceeds a preset level.

9. The device of claim 7 wherein the controller is arranged to calculate the temperature of the extra-oral LED.

10. The device of claim 1 wherein the controller is either: a remote unit separate from the head set; or directly connected to the head set.

11. The device of claim 1 wherein the controller is arranged to direct the extra-oral LED array to emit light at a predetermined rate, frequency, energy density, intensity and duration according to a prescribed treatment regimen.

12. The light therapy device of Claim 1 wherein the extra-oral LED array is capable of emitting light at wavelengths between about 620 to about 680 nm and between about 820 to about 890 nm.

13. The light therapy device of Claim 1 further comprising thermal cooling components comprising a heat sink (51), the thermal cooling components being configured for forced air or liquid cooling.

14. The light therapy device of Claim 1 wherein the extra-oral LED array further comprises optics or reflectors encapsulated in plastic that are capable of directing the light emitted from the LED array.

15. The light therapy device of claim 1 wherein the head set comprises an adjustable strap adapted to fit around the crown of the patient's head for securing the extra-oral light therapy device.

16. The light therapy device of claim 1, comprising a plurality of extra-oral LED arrays.

17. The light therapy device of claim 1 wherein the extra-oral LED array is rotatable between a sagittal axis and a vertical axis.

18. The light therapy device of claim 12, wherein the LED array comprises one or more LEDs.

19. The light therapy device of claim 2, wherein the form-fitting arms (27) are adapted to fit above and around the patient's ears without covering the ears.

20. The light therapy device of claim 1 wherein the frame is composed of a material that allows for flexibility and customization of the frame for differing patient face morphology.

21. The light therapy device of claim 1 wherein the extra-oral LED array is attached by inserting a male portion (6) of the extra-oral LED array into a female portion (8) of the frame.

22. The light therapy device of claim 1 wherein the frame allows for the extra-oral LED array to be attached at more than one position relative to the frame.

23. The light therapy device of claim 1 wherein the extra-oral LED array irradiates light in an amount that is effective for the treatment of a bone disorder or for the biostimulation of bone or soft tissue.

24. The device of claim 1 wherein the extra-oral LED array is adapted to contact a side of the patient's face when the device is worn by the patient.

25. The device of claim 1, further comprising another extra-oral LED array, wherein one extra-oral LED array is adapted to contact the right side of the patient's face when the device is worn by the patient, and the other extra-oral LED array is adapted to contact the left side of the patient's face when the device is worn by the patient.

26. The device of claim 16 wherein a hinge-like member is integrated in the head set between the plurality of LED arrays.

## Patentansprüche

1. Extraorales Lichttherapiegerät (2) für die Behandlung von Kieferknochenerkrankungen und Kieferosteonekrose und Biostimulation von Knochen und Weichgewebe, das Folgendes umfasst:
eine extraorale Halterung (5), wobei die extraorale Halterung Folgendes umfasst:
ein Headset (17) und einen Rahmen (29), der geeignet ist, an den Rücken der Nase eines Patienten angepasst zu werden und über dem Gesicht des Patienten zu liegen, wenn das Gerät von dem Patienten getragen wird;
mindestens einen extraoralen Leuchtdioden ("LED")-Array (4), der abnehmbar mit dem Rahmen über ein Verbindungsstück (21) verbunden ist, der geeignet ist,
mit dem Gesicht des Patienten am Kieferknochen des Patienten in Kontakt zu kommen, wenn das Gerät von dem Patienten getragen wird, und der geeignet ist,
Licht durch das Gesicht des Patienten zum Kieferknochen und Weichgewebe des Patienten auszustrahlen, wenn das Gerät verwendet wird; und
eine programmierbare Steuereinheit (15) zum Steuern des extraoralen Lichttherapiegeräts.

2. Extraorales Lichttherapiegerät nach Anspruch 1, worin das Headset anpassbare Arme (27) umfasst, die sich an die Ohren eines Patienten anpassen.

3. Extraorales Lichttherapiegerät nach Anspruch 1, worin das Verbindungsstück den mindestens einen extraoralen LED-Array (19) an den Rahmen durch Klemmen des mindestens einen extraoralen LED-Arrays an einen Stab oder eine Stange (33), der/die an dem Verbindungsstück befestigt ist, abnehmbar befestigt.

4. Extraorales Lichttherapiegerät nach Anspruch 1, worin der mindestens eine extraorale LED-Array entlang einer horizontalen Achse und einer vertikalen Achse einstellbar ist.

5. Extraorales Lichttherapiegerät nach Anspruch 1, worin der mindestens eine extraorale LED-Array eine Vielzahl von dickschichtigen metallkeramischen LED-Wafers und ein Metallsubstrat zur Übertragung von erzeugter Wärme auf einen Aluminium-Kühlkörper umfasst.

6. Lichttherapiegerät nach Anspruch 1, worin die programmierbare Steuereinheit mit einem vorgeschriebenen Behandlungsregime eines Patienten programmiert ist.

7. Lichttherapiegerät nach Anspruch 1, worin die programmierbare Steuereinheit Schalttechnik umfasst, die zur Überwachung von Veränderungen des Stroms oder der Spannung konfiguriert ist.

8. Lichttherapiegerät nach Anspruch 7, worin die Steuereinheit eine fehlersichere Schalttechnik umfasst, die den Strom und das Licht abschaltet, falls die Wärme in dem Gerät eine voreingestellte Höhe übersteigt.

9. Gerät nach Anspruch 7, worin die Steuereinheit angeordnet ist, die Temperatur der extraoralen LED zu berechnen.

10. Gerät nach Anspruch 1, worin die Steuereinheit eines von Folgenden ist: eine Ferneinheit, die vom Headset getrennt ist; oder eine Einheit, die direkt mit dem Headset verbunden ist.

11. Gerät nach Anspruch 1, worin die Steuereinheit angeordnet ist, den extraoralen LED-Array zur Emission von Licht bei einer vorbestimmten Rate, Frequenz, Energiedichte, Intensität und Dauer entsprechend eines vorgeschriebenen Behandlungsregimes anzuweisen.

12. Lichttherapiegerät nach Anspruch 1, worin der extraorale LED-Array geeignet ist, Licht bei Wellenlängen zwischen etwa 620 bis etwa 680 nm und zwischen etwa 820 bis etwa 890 nm zu emittieren.

13. Lichttherapiegerät nach Anspruch 1, das weiter thermische Kühlkomponenten, die einen Kühlkörper (51) umfassen, umfasst, wobei die thermischen Kühlkomponenten zur forcierten Luft- oder Flüssigkeitskühlung konfiguriert sind.

14. Lichttherapiegerät nach Anspruch 1, worin der extraorale LED-Array weiter in Kunststoff eingebettete Optik oder Reflektoren umfasst, die geeignet sind, das Licht, das vom LED-Array emittiert wird, zu lenken.

15. Lichttherapiegerät nach Anspruch 1, worin das Headset ein verstellbares Band umfasst, das geeignet ist, um den Kopfumfang des Patienten zur Befestigung des extraoralen Lichttherapiegeräts angepasst zu werden.

16. Lichttherapiegerät nach Anspruch 1, das eine Vielzahl von extraoralen LED-Arrays umfasst.

17. Lichttherapiegerät nach Anspruch 1, worin der extraorale LED-Array zwischen einer Sagittalachse und einer vertikalen Achse rotierbar ist.

18. Lichttherapiegerät nach Anspruch 12, worin der LED-Array eine oder mehrere LEDs umfasst.

19. Lichttherapiegerät nach Anspruch 2, worin die anpassbaren Arme (27) geeignet sind, über den Ohren und um die Ohren des Patienten herum zu liegen, ohne die Ohren zu bedecken.

20. Lichttherapiegerät nach Anspruch 1, worin der Rahmen aus einem Material besteht, das Flexibilität und Anpassung des Rahmens an eine unterschiedliche Morphologie des Gesichts eines Patienten ermöglicht.

21. Lichttherapiegerät nach Anspruch 1, worin der extraorale LED-Array durch Einstecken eines männlichen Teils (6) des extraoralen LED-Arrays in einen weiblichen Teil (8) des Rahmens befestigt wird.

22. Lichttherapiegerät nach Anspruch 1, worin der Rahmen ermöglicht, dass der extraorale LED-Array an mehr als einer Position bezüglich des Rahmens befestigt werden kann.

23. Lichttherapiegerät nach Anspruch 1, worin der extraorale LED-Array Licht in einer Menge ausstrahlt, die für die Behandlung einer Knochenerkrankung oder für die Biostimulation von Knochen oder Weichgewebe wirksam ist.

24. Gerät nach Anspruch 1, worin der extraorale LED-Array geeignet ist, mit einer Seite des Gesichts eines Patienten in Kontakt zu kommen, wenn das Gerät von dem Patienten getragen wird.

25. Gerät nach Anspruch 1, das weiter einen anderen extraoralen LED-Array umfasst, worin ein extraoraler LED-Array geeignet ist, mit der rechten Seite des Gesichts des Patienten in Kontakt zu kommen, wenn das Gerät von dem Patienten getragen wird, und der andere extraorale LED-Array geeignet ist, mit der linken Seite des Gesichts des Patienten in Kontakt zu kommen, wenn das Gerät von dem Patienten getragen wird.

26. Gerät nach Anspruch 16, worin ein scharnierartiges Element in das Headset zwischen die Vielzahl von LED-Arrays eingebaut ist.

## Revendications

1. Dispositif extra-oral de thérapie par la lumière (2) pour le traitement des troubles osseux des mâchoires et de l'ostéonécrose des mâchoires ainsi que pour la biostimulation des os et des tissus mous, comprenant :
un support extra-oral (5), le support extra-oral comprenant un casque (17) et un cadre (29) adaptés pour se placer sur l'arête du nez d'un patient et reposer pardessus le visage du patient quand celui-ci porte le dispositif ;
au moins un réseau de diodes électroluminescentes ("DEL") extra-oral (4) qui est connecté de façon amovible au cadre par l'intermédiaire d'un connecteur (21),
lequel est adapté pour faire contact avec le visage du patient sur la mâchoire du patient quand celui-ci porte le dispositif, et lequel est adapté pour rayonner une lumière à travers le visage du patient vers la mâchoire et les tissus mous du patient quand le dispositif est en service ; et
un contrôleur programmable (15) pour commander le dispositif extra-oral de thérapie par la lumière.

2. Dispositif extra-oral de thérapie par la lumière selon la revendication 1, dans lequel le casque comprend des bras ajustables (27) qui épousent la forme des oreilles d'un patient.

3. Dispositif extra-oral de thérapie par la lumière selon la revendication 1, dans lequel le connecteur attache de façon amovible l'au moins un réseau de DEL extra-oral (19) au cadre en accrochant l'au moins un réseau de DEL extra-oral à une barre ou tige (33) fixée au connecteur.

4. Dispositif extra-oral de thérapie par la lumière selon la revendication 1, dans lequel l'au moins un réseau de DEL extra-oral est réglable le long d'un axe horizontal et d'un axe vertical.

5. Dispositif extra-oral de thérapie par la lumière selon la revendication 1, dans lequel l'au moins un réseau de DEL extra-oral comprend une pluralité de tranches de DEL céramo-métalliques en couches épaisses et un substrat métallique pour transférer la chaleur produite vers un dissipateur thermique en aluminium.

6. Dispositif de thérapie par la lumière selon la revendication 1, dans lequel le contrôleur programmable est programmé avec le traitement prescrit au patient.

7. Dispositif de thérapie par la lumière selon la revendication 1, dans lequel le contrôleur programmable comprend des circuits configurés pour contrôler les changements de courant ou de tension.

8. Dispositif de thérapie par la lumière selon la revendication 7, dans lequel le contrôleur comprend des circuits de sécurité qui coupent le courant et la lumière si la chaleur dans le dispositif dépasse un niveau préréglé.

9. Dispositif selon la revendication 7, dans lequel le contrôleur est agencé pour calculer la température des DEL extra-orales.

10. Dispositif selon la revendication 1, dans lequel le contrôleur est soit : une unité distante du casque ; soit une unité connectée directement au casque.

11. Dispositif selon la revendication 1, dans lequel le contrôleur est agencé pour diriger le réseau de DEL extra-oral afin d'émettre une lumière à une cadence, fréquence, densité d'énergie, intensité et durée prédéterminées en fonction d'un traitement prescrit.

12. Dispositif de thérapie par la lumière selon la revendication 1, dans lequel le réseau de DEL extra-oral est capable d'émettre une lumière à des longueurs d'onde comprises entre environ 620 à environ 680 nm et entre environ 820 à environ 890 nm.

13. Dispositif de thérapie par la lumière selon la revendication 1, comprenant en outre des composants de refroidissement thermique comprenant un dissipateur thermique (51), les composants de refroidissement thermique étant configurés pour un refroidissement par air ou liquide forcé.

14. Dispositif de thérapie par la lumière selon la revendication 1, dans lequel le réseau de DEL extra-oral comprend en outre des optiques ou réflecteurs encapsulés dans du plastique et capables de diriger la lumière émise depuis le réseau de DEL.

15. Dispositif de thérapie par la lumière selon la revendication 1, dans lequel le casque comprend une sangle ajustable adaptée pour se placer autour du crâne du patient afin de fixer le dispositif extra-oral de thérapie par la lumière.

16. Dispositif de thérapie par la lumière selon la revendication 1, comprenant une pluralité de réseaux de DEL extra-oraux.

17. Dispositif de thérapie par la lumière selon la revendication 1, dans lequel le réseau de DEL extra-oral est rotatif entre un axe sagittal et un axe vertical.

18. Dispositif de thérapie par la lumière selon la revendication 12, dans lequel le réseau de DEL comprend une ou plusieurs DEL.

19. Dispositif de thérapie par la lumière selon la revendication 2, dans lequel les bras ajustables (27) sont adaptés pour se placer au-dessus et autour des oreilles du patient sans recouvrir celles-ci.

20. Dispositif de thérapie par la lumière selon la revendication 1, dans lequel le cadre est composé d'un matériau qui confère souplesse et possibilité de personnalisation du cadre pour différentes morphologies de visage de patient.

21. Dispositif de thérapie par la lumière selon la revendication 1, dans lequel le réseau de DEL extra-oral est attaché en insérant une partie mâle (6) du réseau de DEL extra-oral dans une partie femelle (8) du cadre.

22. Dispositif de thérapie par la lumière selon la revendication 1, dans lequel le cadre permet d'attacher le réseau de DEL extra-oral à plus d'une position par rapport au cadre.

23. Dispositif de thérapie par la lumière selon la revendication 1, dans lequel le réseau de DEL extra-oral rayonne une lumière en une quantité efficace pour le traitement d'un trouble osseux ou pour la biostimulation des os ou des tissus mous.

24. Dispositif selon la revendication 1, dans lequel le réseau de DEL extra-oral est adapté pour faire contact avec un côté du visage du patient quand le dispositif est porté par le patient.

25. Dispositif selon la revendication 1, comprenant en outre un autre réseau de DEL extra-oral, dans lequel un réseau de DEL extra-oral est adapté pour faire contact avec le côté droit du visage du patient quand le patient porte le dispositif, et l'autre réseau de DEL extra-oral est adapté pour faire contact avec le côté gauche du visage du patient quand le patient porte le dispositif.

26. Dispositif selon la revendication 16, dans lequel un élément du type charnière est intégré au casque entre la pluralité de réseaux de DEL.
